# EUROPEAN PATENT SPECIFICATION

(11) **EP 2 357 247 B1**
(45) Date of publication and mention of the grant of the patent: **09.03.2016**
(21) Application number: 09831649.0
(22) Date of filing: 02.12.2009
(51) Int. Cl.: C12P 7/62, C12N 15/09, C12R 1/01, C12R 1/05

(54) **METHOD FOR PRODUCING POLY-3-HYDROXYALKANOATE**
VERFAHREN ZUR HERSTELLUNG VON POLY-3-HYDROXYALKANOAT
PROCÉDÉ DE FABRICATION DE POLY-3-HYDROXYALCANOATE

(30) Priority: 09.12.2008 JP 2008313329
(43) Date of publication of application: 17.08.2011
(73) Proprietor: Kaneka Corporation, Osaka (JP)
(72) Inventor: ASAI, Yousuke, Takasago-shi, Hyogo 676-8688 (JP); UENO, Masakuni, Takasago-shi, Hyogo 676-8688 (JP); TAKITA, Masaki, Takasago-shi, Hyogo 676-8688 (JP)
(74) Representative: Isarpatent
(86) International application number: PCT/JP2009/006544
(87) International publication number: WO 2010/067541

(56) References cited:
- EP-A2- 0 520 405
- WO-A1-2004/033700
- WO-A1-2004/065608
- JP-A- 2008 054 541
- JP-A- 2008 086 238
- JP-T- 2000 502 399
- US-B1- 6 645 743

## Description

### [Technical Field]

The present invention relates to a method for forming poly-3-hydroxyalkanoic acid agglomerates from an aqueous suspension containing poly-3-hydroxyalkanoic acid.

### [Background Art]

Poly-3-hydroxyalkanoic acid (hereinafter, abbreviated as PHA) is a thermoplastic polyester produced and accumulate in cells of many microorganism species as an energy storage material, and has biodegradability. At present, non-petroleum plastics have attracted attention owing to increased environmental consciousness. In particular, biodegradable plastics such as PHA which are incorporated in material recycling in the natural world and thus the degradation products do not become harmful have drawn attention, and to put them into practical applications has been desired. Particularly, since PHA formed and accumulated by microorganisms in cellular bodies is incorporated into the process of carbon cycle of the natural world, lower adverse effects on the ecological system have been expected.

Since PHA produced by a microorganism usually forms a granular body and is accumulated in the cellular bodies of the microorganism, a step of separating and recovering PHA from inside the cellular bodies of the microorganism is necessary for utilizing PHA as a plastic. In addition, for using PHA as a plastic, it is desired to increase the purity of PHA, and to lower the content of contaminants of constitutive components and the like of cellular bodies, and the like.

As a method for degradation and/or removal of components other than PHA derived from an organism, a method in which components other than PHA derived from an organism are solubilized and removed by a physical treatment, a chemical treatment or a biological treatment was proposed. For example, a method in which a treatment of disrupting cellular bodies of a PHA-containing microorganism and a treatment with a surfactant are combined (Patent Document 1), a method in which a heat treatment after adding an alkali is followed by carrying out a disruption treatment (Patent Document 2), and the like may be exemplified. In addition, a method for obtaining PHA in which aqueous suspension of cellular bodies of a microorganism is subjected to a treatment with sodium hypochlorite or an enzyme to solubilize components other than PHA derived from an organism (Patent Document 3) was also proposed.

Also, as a means for recovering PHA from an aqueous suspension obtained by disrupting cellular bodies of a PHA-containing microorganism or solubilizing components other than PHA derived from an organism, separating operation such as centrifugation or filtration, or drying operation such as spray drying may be exemplified. However, when PHA particles produced by cellular bodies are directly recovered as primary particles, fine powders increase, and thus a problem of handling as a product to be difficult may be involved.

It is generally known that addition of a salt or the like enables solid powders in a fine slurry solid-liquid dispersion liquid to be aggregated. However, it is extremely difficult to allow only target PHA to be aggregated from an aqueous suspension containing cellular components leaked from disrupted cells, such as proteins in addition to PHA, and there has been no example of such findings. Even if aluminum sulfate, which has been widely used in activated sludge treatments, etc. , or the like is used, it is impossible to allow only target PHA to be selectively aggregated since almost all components in the aqueous suspension are aggregated. In addition, even if PHA can be selectively aggregated with a polymeric coagulant or the like, quality as a polymer material may be affected since separating these additives from PHA is difficult.

As a method conducted without using a coagulant, a method in which a PHA suspension is heated (Patent Document 4), a method in which heating and cooling are repeated (Patent Document 5), and the like have been known. In any of the methods, lowering of the molecular weight of PHA upon heating has been concerned since heating to around the melting point of PHA is carried out.

On the other hand, a method in which after PHA is dissolved in an organic solvent, an organic solvent having low solubility or water is added thereto to allow thus dissolved PHA to be deposited has been known. Since a PHA solution can be purified according to this method, it has enabled to obtain PHA having a highest purity. As such a solvent extraction method, an example in which a lower ketone or the like is used as an extraction solvent (Patent Document 6), an example in which tetrahydrofuran is used (Patent Document 7) and the like were reported. If a poor solvent is added to an organic solvent including PHA dissolved therein, deposition of PHA is enabled, and it has been possible to comparatively arbitrarily control the shape and size of the deposit, depending on the solvent to be added, and conditions of addition such as a temperature and amount of addition, as well as stirring conditions during the addition, and the like.

The capability of controlling the shape and size of the deposited matter by thus allowing PHA to be deposited from an organic solvent has been very advantageous in view of problems of PHA purified using a water soluble solvent that it includes a large amount of fine powders. However, this process has involved fundamental problems of: use of a large quantity of organic solvent in extraction; lowering of the molecular weight of PHA during the purification step as PHA originally being highly degradable is heated for dissolving the same; and the like.

Accordingly, when PHA produced by a microorganism is industrially separated and purified, there has been problems of failure in obtaining PHA particles having an arbitrary volume mean particle diameter with favorable productivity while decreasing contaminants derived from constitutive components of cellular bodies, taking into consideration the environmental aspects. Furthermore, since the parameter dominating over aggregation of PHA particles has been unclear, it has been still further difficult to propose means for solving these problems.

US 2006084161 A1 describes a method for separating and purifying a PHA without causing a serious decrease of the molecular weight to obtain a highly pure PHA in a high yield, which comprises efficiently removing cell components other than PHA particles from a cultured PHA-containing microbial cell. US2006084161A1 does neither disclose the measurement of the nitrogen content using ninhydrin reaction or the adjustment of the nitrogen content to below 1500 ppm.

EP 1550724 A1 describes a method for agglomerating a poly-3-hydroxyalkanoic acid which comprises suspending particles of the poly-3-hydroxyalkanoic acid in a hydrophilic solvent or a mixture comprising water and a hydrophilic solvent, and stirring the obtained suspension at a temperature not more than the boiling point of said suspension. EP 1550724 A1 is silent about the nitrogen content.

### [Prior Art Document]

### [Patent Document]

Patent Document 1: JP-T No. H08-502415
Patent Document 2: PCT International Publication No. 2004/065608
Patent Document 3: JP-A No. 2005-348640
Patent Document 4: JP-T No. 2000-502399
Patent Document 5: JP-T No. 2002-517582
Patent Document 6: JP-T No. H10-504460
Patent Document 7: JP-A No. H07-79788

### [SUMMARY OF THE INVENTION]

### [Problems to be Solved by the Invention]

Problems to be solved by the present invention is, when industrially separating and purifying PHA produced by a microorganism, to obtain PHA particles having an arbitrary volume mean particle diameter with favorable productivity and with decreased amount of an organic solvent used while decreasing contaminants derived from constitutive components of cellular bodies, without adding a salt, a polymeric coagulant or the like, and also without carrying out a high temperature treatment.

### [Means for Solving the Problems]

The inventors found that PHA is aggregated without addition of a salt, a polymeric coagulant or the like, at a temperature lower than the melting point without heating to around the melting point of PHA, by decreasing the amount of organic nitrogen in an aqueous suspension containing PHA. Accordingly, the present invention was accomplished.

The present invention realtes to a method for producing agglomerates of poly-3-hydroxyalkanoic acid comprising:
culturing a microorganism having the ability to intracellularly produce poly-3-hydroxyalkanoic acid;
disrupting the microorganism containing the poly-3-hydroxyalkanoic acid;
purifying poly-3-hydroxyalkanoic acid by degradation and/or removal of impurities to obtain an aqueous suspension containing poly-3-hydroxyalkanoic acid having an amount of organic nitrogen in the aqueous suspension containing poly-3-hydroxyalkanoic acid adjusted to not greater than 1,500 ppm per weight of poly-3-hydroxyalkanoic acid as determined by ninhydrin reaction; and
thereafter allowing poly-3-hydroxyalkanoic acid to be aggregated in the aqueous suspension, thereby obtaining agglomerates of poly-3-hydroxyalkanoic acid.

According to the present invention, a solvent included in the aqueous suspension containing poly-3-hydroxyalkanoic acid preferably contains water, an organic solvent that is miscible with water, or a mixed solvent of water and the organic solvent.

According to the present invention, poly-3-hydroxyalkanoic acid is preferably a copolymer constituted with two or more types of 3-hydroxyalkanoic acid selected from the group consisting of 3-hydroxypropionate, 3-hydroxybutyrate, 3-hydroxyvalerate, 3-hydroxyhexanoate, 3-hydroxyheptanoate, and 3-hydroxyoctanoate.

According to the present invention, poly-3-hydroxyalkanoic acid is preferably a binary copolymer of 3-hydroxyhexanoate and 3-hydroxybutyrate, or a ternary copolymer of 3-hydroxyhexanoate, 3-hydroxybutyrate and 3-hydroxyvalerate.

According to the present invention, poly-3-hydroxyalkanoic acid is preferably poly-3-hydroxyalkanoic acid produced by a microorganism that yields poly-3-hydroxyalkanoic acid.

According to the present invention, the microorganism that yields poly-3-hydroxyalkanoic acid is preferably a microorganism belonging to genus Aeromonas, genus Alcaligenes, genus Ralstonia, or genus Cupriavidus.

According to the present invention, the microorganism that yields poly-3-hydroxyalkanoic acid is preferably Cupriavidus necator.

According to the present invention, the microorganism that yields poly-3-hydroxyalkanoic acid is preferably a transformant into which a poly-3-hydroxyalkanoic acid synthase gene derived from Aeromonas caviae and/or a variant thereof was introduced.

According to the present invention, the amount of organic nitrogen in an aqueous suspension containing poly-3-hydroxyalkanoic acid is preferably adjusted to not greater than 600 ppm per weight of poly-3-hydroxyalkanoic acid.

### [Effects of the Invention]

According to the present invention, PHA yielded by a microorganism can be purified not by an extraction operation with an organic solvent, and aggregation of PHA is enabled at a temperature lower than the melting point of PHA without adding a third component such as a salt or a polymeric coagulant. PHA agglomerates with a fewer fine powders can be obtained with superior productivity while preventing contamination with constitutive components of cellular bodies. Thus obtained PHA agglomerates do not necessitate concerns about influences on quality which may be caused by adding a third substance, and lowering of the molecular weight of PHA by heating can be avoided.

### [Mode for Carrying Out the Invention]

The microorganism for use in the present invention is not particularly limited as long as is a microorganism that intracellularly produces PHA. A microorganism isolated from natural sources, a microorganism deposited with Microorganism Depositary (for example, IFO, ATCC, etc.), a variant or a transformant which can be prepared therefrom, or the like may be used. For example, bacteria of genus Cupriavidus, genus Alcaligenes, genus Ralstonia, genus Pseudomonas, genus Bacillus, genus Azotobacter, genus Nocardia, and genus Aeromonas, and the like may be involved. Of these, a microorganism belongs to genus Aeromonas, genus Alcaligenes, genus Ralstonia, or genus Cupriavidus is preferred. In particular, a strain of Alcaligenes Lipolytica (A. lipolytica), Alcaligenes Latus (A. latus), Aeromonas Caviae (A. caviae), Aeromonas Hydrophila (A. Hydrophila), Cupriavidus necator (C. Necator) or the like is more preferred, and Cupriavidus necator is most preferred. Also, when the microorganism does not originally have an ability to produce PHA or produces only a small amount of PHA, a synthase gene of intended PHA and/or a variant thereof may be introduced into the microorganism, and the resulting transformant may be used. Although the synthase gene of PHA which may be used in producing such a transformant is not particularly limited, a PHA synthase gene derived from Aeromonas caviae is preferred. By culturing these microorganisms under appropriate conditions, cellular bodies of a microorganism including PHA accumulated in cellular bodies areobtained. Although the culture process is not particularly limited, for example, a process disclosed in JP-A No. H05-93049 or the like may be used.

PHA in the present invention is a generic name of a polymer constituted with 3-hydroxyalkanoic acid as a monomer unit. Although the constituting 3-hydroxyalkanoic acid is not particularly limited, specifically, a copolymer of 3-hydroxybutyrate (3HB) and other 3-hydroxyalkanoic acid, a copolymer of 3-hydroxyalkanoic acid including 3-hydroxyhexanoate (3HH), or the like may be exemplified. Furthermore, copolymers of two or more types of 3-hydroxyalkanoic acid selected from the group consisting of 3-hydroxypropionate, 3-hydroxybutyrate, 3-hydroxyvalerate, 3-hydroxyhexanoate, 3-hydroxyheptanoate and 3-hydroxyoctanoate as monomer units may be also exemplified. Among these, copolymers including 3HH as a monomer unit, for example, a binary copolymer (PHBH) of 3HB and 3HH (Macromolecules, 28, 4822-4828 (1995)), or a ternary copolymer (PHBVH) of 3HB, 3-hydroxyvalerate (3HV) and 3HH (Japanese Patent No. 2,777,757, JP-A No. H08-289797) are more preferred in light of physical properties of the resulting polyester. Herein, the composition ratio of each monomer unit constituting the binary copolymer of 3HB and 3HH, i.e., PHBH is not particularly limited; however, a composition ratio of 3HH unit being 1 to 99 mol%, preferably 1 to 50 mol%, and more preferably 1 to 25 mol% is suited, provided that the sum total of the entire monomer units is 100 mol%. In addition, the composition ratio of each monomer unit constituting the ternary copolymer of 3HB, 3HV and 3HH, i.e., PHBVH is not particularly limited; however, composition ratios suitably fall within the range of, for example, 3HB unit of 1 to 95 mol%, 3HV unit of 1 to 96 mol%, and 3HH unit of 1 to 30 mol%, respectively, provided that the sum total of the entire monomer units is 100 mol%.

Before degradation and/or removal of impurities of components other than PHA derived from an organism, and the like in the present invention,the cells containing PHA are disrupted beforehand by a physical treatment, a chemical treatment or a biological treatment. Accordingly, a degradation and/or removal step that will follow can be efficiently performed. Although the disruption process is not particularly limited, any process carried out using fluid shearing force or solid shearing force, or by grinding, by means of a conventionally well-known French press, homogenizer, X-press, ball mill, colloid mill, DYNO mill, ultrasonic homogenizer or the like may be employed. Alternatively, a process in which an agent such as an acid, alkali, surfactant, organic solvent, cell wall synthesis inhibitor or the like is used, a process in which an enzyme such as lysozyme, pectinase, cellulase or zymolyase is used, a process in which supercritical fluid is used, an osmotic disruption process, a freezing process, a dry disruption process, and the like may be exemplified. Also, an autolysis process carried out using an action of protease, esterase, etc., included in the cells per se is also exemplified as one type of disruption process. In the foregoing disruption process, to select a process capable of inhibiting lowering of the molecular weight of PHA by a series of treatments is desired. In addition, these disruption processes may be used either alone, or a plurality of the processes may be used in combination. Also, either batchwise processing, or continuous processing may be conducted.

In general, an aqueous PHA suspension prepared by disrupting the PHA-containing cellular bodies according to the aforementioned process is contaminated with proteins, nucleic acids, lipids and sugar components in cells, and other constitutive components of cellular bodies, culture substrate residues, and the like. It is preferred to carry out a dehydration step for separating water containing these proteins and the like prior to the degradation and/or removal step described in the following. Accordingly, the amount of impurities included in the aqueous PHA suspension can be reduced, and thus the degradation and/or removal step can be efficiently carried out. Although dehydration process is not particularly limited, process of filtration, centrifugal separation, or precipitation separation may be exemplified. The concentration of PHA in the aqueous suspension subjected to the degradation and/or removal step is not particularly limited, which is preferably not less than 50 g/L, more preferably not less than 100 g/L, still more preferably not less than 200 g/L, and even more preferably not less than 300 g/L. In addition, the aforementioned dehydration step may be performed for the purpose of adjusting the concentration of PHA in the aqueous suspension.

The process of degradation and/or removal of impurities such as components other than PHA derived from the organism is not particularly limited, and for example, a process carried out using an enzyme may be exemplified. The enzyme which may be used includes a proteolytic enzyme, a lipolytic enzyme, cell wall degrading enzyme, nucleolytic enzyme, and the like. Specific examples of these enzymes include the followings. These may be used either alone, or two or more of these may be used in combination.
(1) Proteolytic enzyme
   Esperase, Alcalase, pepsin, trypsin, papain, chymotrypsin, aminopeptidase, carboxypeptidase, and the like
(2) Lipolytic enzyme
   lipase, phospholipase, cholineesterase, phosphatase, and the like
(3) Cell wall degrading enzyme
   lysozyme, amylase, cellulase, maltase, saccharase, α-glycosidase, β-glycosidase, N-glycosidase, and the like
(4) Nucleolytic enzyme
   ribonuclease, deoxyribonuclease, and the like

The enzyme used in degradation of impurities such as components other than PHA derived from the organism is not limited to those described above, and may be an arbitrary enzyme having an activity of degradation of components derived from the organism as long as it can be used in industrial products. Also, a commercially available enzyme detergent used for washing or the like in general may be also used. Still further, an enzyme composition containing, for example, a stabilizing agent of an enzyme, an antisoil redeposition agent, etc., and the enzyme is also acceptable, and it is not necessarily limited to use of only an enzyme. Preferable proteolytic enzymes which may be industrially used include, among the above-illustrated enzymes, protease A, protease P, protease N (all manufactured by Amano Enzyme inc.), Esperase, Alcalase, Savinase, Everlase (all manufactured by Novozymes A/S), and the like, and these can be suitably used also in light of the degradation activity, but not limited thereto.

The enzyme treatment is preferably carried out until a desired degree of the treatment is achieved, and the time period is usually 0.5 to 2 hrs. The amount of the enzyme to be used depends on the type and activity of the enzyme, and is not particularly limited, which is preferably 0.001 to 10 parts by weight, and in light of the cost, more preferably 0.001 to 5 parts by weight relative to 100 parts by weight of PHA.

Other process for the degradation of impurities such as components other than PHA derived from the organism includes a process in which hypochlorous acid or hydrogen peroxide is used. When hypochlorous acid is used, the pH of the system is adjusted to fall within an alkaline region, and the degradation is executed under conditions in which heat, light, or contact with metal can be inhibited, whereby PHA having a low amount of remaining chlorine can be obtained. The pH is desirably not less than 8, more desirably not less than 10, and still more desirably not less than 12. The treatment temperature is desirably not greater than 40°C, more desirably not greater than 30°C, still more desirably not greater than 20°C, and for surely achieving the effects, the treatment is carried out at not greater than 10°C.

As described above, in the aforementioned dehydration step, for separating PHA from water containing impurities such as other components derived from the organism, filtration, centrifugal separation or the like may be carried out. Although the filtration process is not particularly limited, a process carried out using Nutsche or the like, or process such as suction filtration or pressure filtration is desired. For industrial applications, filtration equipment having a compressing function such as a filter press, tube press, plate press, gauge press, belt press, screw press or disk press, as well as a centrifugal dehydrator, a multiple cylindrical filtration element or the like may be selected. When improving productivity is intended, continuous type such as a multiple cylindrical filtration element is desired. As a process for removing scums of particles in a continuous type filtration element, a string system, a scraper system, a precoating scraper system or the like may be involved. Alternatively, a membrane separation system may be also employed. As a process for filtration involving membrane separation, dead end filtration, or cloth flow filtration may be selected. Any case may be selected based on the filterability, the extent of clogging of the filter material, membrane and the like. In addition, reduced pressure or vacuum may be provided, or compression may be permitted. Furthermore, a process in which centrifugal force is employed may be used. As a filter material, any of a variety of materials such as a paper, woven fabric, nonwoven fabric, screen, sintered plate, unglazed pottery, polymer membrane, punching metal or wedge wire may be selected. Any one may be selected depending upon the productivity and degree of clogging and the like. Also, a filter aid may or may not be used. When a filter aid is used, either a process of precoating the filter aid onto the filter material beforehand (i.e., precoating system), or a process of previously adding to a liquid subjected to the filtration (i.e., body feeding method) may be employed.

Although the process of centrifugal separation in the aforementioned dehydration step is not particularly limited, a centrifugal settler, a centrifugal dehydrator or the like may be used. In the case of a centrifugal settler, a separator type, a cylindrical type, and a decanter type may be exemplified. In the case of the separator type, a disk type, a self cleaning type, a nozzle type, a screw decanter type, a skimming type, and the like may be exemplified. Depending on the procedure of discharging precipitated components, there are batch type and continuous type, respectively. Also, with respect to the centrifugal dehydrator, there may be batch type and continuous type. Separation of precipitates containing PHA from culture liquid components is enabled with these equipments, based on the difference in specific gravity.

Other process which may be used in the above dehydration step may include a floatation process, an electrophoresis process, a cyclone processing, and the like. The processes of filtration and centrifugal separation, as well as floatation may be used alone, or in combination.

After PHA was recovered by the process such as filtration and/or centrifugal separation in the aforementioned dehydration step, the recovered PHA is washed with water or the like, whereby further purified PHA can be obtained. The washing may be carried out using not only water but also an organic solvent, and water and an organic solvent may be used as a mixture. Also, the pH of water may be adjusted. When an organic solvent is used as a washing solvent, preferably, a hydrophilic solvent, and more specifically methanol, ethanol, acetone, acetonitrile, tetrahydrofuran, a ketone, an amine or the like may be used. In addition, a surfactant or the like may be added to water. A plurality of types of these organic solvents and water may be used as a mixture. Moreover, water or the organic solvent may be heated or sprayed in the form of vapor to improve the washing property as long as this process is carried out within a short period of time.

As explained in the foregoing, according to the most suitable aspect of the present invention, agglomerates of PHA can be efficiently produced by sequentially carrying out: a culture step of culturing a microorganism having an ability to intracellularly produce PHA; a disruption step of disrupting the microorganism containing PHA; a dehydration step of separating water from an aqueous suspension containing thus disrupted microorganism; a purification step of degradation and/or removal of impurities; a washing step of washing PHA; and aggregation step of allowing PHA to be aggregated in the resulting aqueous PHA suspension to obtain PHA agglomerates. However, the present invention does not necessarily require carrying out all the steps described above.

By carrying out the purification step of degradation and/or removal of impurities, and/or the washing step of washing PHA as described above to decrease the amount of constitutive components of cellular bodies in the aqueous suspension containing PHA beforehand, aggregation of PHA to recover the same is enabled at comparatively low temperatures, without need of a process of adding a salt or a polymeric coagulant, or heating at high temperatures. Conditions desired for aggregation of PHA may be represented in terms of the amount of organic nitrogen per weight of PHA in the aqueous PHA suspension. The amount of the organic nitrogen is not greater than 1,500 ppm. When the amount is greater than 1,500 ppm, aggregation of PHA does not proceed efficiently. The amount is preferably not greater than 1,000 ppm, more preferably not greater than 600 ppm, still more preferably not greater than 400 ppm, even more preferably not greater than 300 ppm, and most preferably not greater than 100 ppm.

The aggregation as used herein means that the volume mean particle diameter of PHA particles becomes at least five times, desirably at least ten times, and more desirably at least 15 times with respect to the volume mean particle diameter of PHA before subjecting to the aggregation operation.

The solvent included in the aqueous suspension in the present invention may include water, an organic solvent that is miscible with water, or a mixed solvent of water and the organic solvent. The organic solvent used may be only one type, or two or more types may be used in combination. In addition, the concentration of the organic solvent in the mixed solvent of water and the organic solvent is not particularly limited as long as it is not beyond the solubility of the organic solvent used in water. Furthermore, although the organic solvent that is miscible with water is not particularly limited, for example, alcohols such as methanol, ethanol, 1-propanol, 2-propanol, 1-butanol, 2-butanol, iso-butanol, pentanol, hexanol and heptanol, ketones such as acetone and methyl ethyl ketone, ethers such as tetrahydrofuran and dioxane, nitriles such as acetonitrile and propionitrile, amides such as dimethylformamide and acetamide, dimethyl sulfoxide, pyridine, piperidine, and the like may be exemplified. Among these, methanol, ethanol, 1-propanol, 2-propanol, 1-butanol, 2-butanol, iso-butanol, acetone, methyl ethyl ketone, tetrahydrofuran, dioxane, acetonitrile, propionitrile and the like are suited in light of favorable removability and the like. Still further, methanol, ethanol, 1-propanol, 2-propanol, 1-butanol, 2-butanol, iso-butanol, acetone and the like are more preferred in light of favorable availability. Still more preferred are methanol, ethanol and acetone. It should be noted that other solvent and/or components derived from the cellular bodies and compounds generated during purification may be contained as long as essential features of the present invention is impaired.

By heating the aqueous PHA suspension from which constitutive components of cellular bodies such as proteins were removed and adjusted to have the amount of the organic nitrogen to be not greater than 1,500 ppm, aggregation of PHA is permitted. The heating temperature in this process is preferably as low as possible in order to inhibit lowering of the molecular weight of PHA. By heating at a temperature is as low as possible, lowering of the molecular weight during the treatment step can be prevented. Specifically, although it may vary depending on each monomer composition, the heating temperature may be lower than the melting point of PHA that is about 130 to 180°C, preferably not greater than 130°C, and more preferably not greater than 110°C. Under alkaline conditions, the heating temperature is more preferably not greater than 90°C, and particularly preferably not greater than 50°C. The lower limit of the heating temperature is not particularly limited; however, it is preferably not less than 20°C for producing agglomerates having a larger particle size. The pH of the aqueous suspension in the aggregation step is not particularly limited, but the pH may fall within the alkali region of 8 or greater. The time period required for elevating the temperature may vary depending on the apparatus size and capacity; however, it is necessary to heat enough until reaching the temperatures at which aggregation of PHA is effected and the particle size increased. The heating time period after reaching the aforementioned heating temperature is about 5 hrs or shorter, preferably 2 hrs or shorter, more preferably 1 hour or shorter, and still more preferably 30 min or shorter. Heating for at least 1 sec or longer is preferred.

Accordingly, PHA can be aggregated and obtained, without adding a third component such as a salt or a polymeric coagulant by previously decreasing the amount of constitutive components of cellular bodies in an aqueous PHA suspension. Also, since adjusting the pH of the aqueous suspension to the acidic range, or heating to a high temperature, leading to concern about lowering of the molecular weight of PHA is not needed upon aggregation of PHA, lowering of the molecular weight of PHA can be prevented, and aggregation of contaminants such as proteins can be inhibited.

### [EXAMPLES]

Hereinafter, the present invention is explained in more detail by way of Examples in the following, but the present invention is not limited only to these Examples.

(Process for Determining Amount of Organic Nitrogen in Aqueous PHA Suspension (per weight of PHA))

The entirety of a water soluble solvent in an aqueous PHA suspension was evaporated to obtain a residual solid content. To this solid content was added 5M NaOH, and a hydrolysis reaction was carried out at 95°C. This hydrolysis liquid was neutralized with the equivalent amount of a 60% aqueous acetic acid solution, and thereto were added an acetate buffer and a ninhydrin solution to allow a color reaction at 100°C. The absorbance of this color reaction liquid was measured with a ratio beam spectrophotometer model U-1800 manufactured by Hitachi, Ltd. By comparing this absorbance with a calibration curve produced using a leucine sample, the amount of organic nitrogen in the solid content was calculated. The amount of organic nitrogen in the aqueous PHA suspension (per weight of PHA) was determined in terms of the amount of organic nitrogen per weight of the solid content.

(Process for Measuring Volume Mean Particle Diameter of PHA Particles in Aqueous PHA Suspension)

The volume mean particle diameter of PHA particles in the aqueous PHA suspension was determined with a laser diffraction scattering particle size distribution meter.

### (Example 1) Preparation of Cell Culture Liquid

Ralstonia eutropha KNK-005 strain disclosed in paragraph No. [0049] of PCT International Publication No. 2008/010296 was cultured according to a process disclosed in paragraph Nos. [0050]-[0053] of the same document to obtain a cell culture liquid including cellular bodies containing PHA. Note that Ralstonia eutropha is classified as Cupriavidus necator at present.

### (Example 2) Sterilization Process

The obtained culture liquid was subjected to a treatment of heating with stirring at an internal temperature of 60 to 80°C for 20 min to execute a sterilization treatment.

### (Example 3) Preparation of Aqueous PHA Suspension

The culture liquid obtained by culturing and subjected to a sterilization operation according to the aforementioned process was subjected to an alkali treatment (adding 30% NaOH to adjust the pH to 11.8, and maintained at a temperature of 50°C for 1 hour while stirring), and thereafter a mechanical disruption treatment (treatment with a homogenizer at high pressure (using model NS3015, Niro Soavi S.P.A), liquid fed seven times at pH of not less than 12.5, at 600 bar) was carried out. To the culture liquid subjected to the disruption treatment was added a protease (manufactured by Novozymes A/S, trade name: Esperase) in a weight of 1/100 the PHA included, and the mixture was maintained at a pH of 10, and an internal temperature of 60°C for 1 hour with stirring. This mixture was subjected to centrifugal separation (1,400 G, 20 min), and the supernatant was removed in part, Subsequently an operation of adding the same amount of pure water to the mixture and suspending PHA was repeated several times, whereby aqueous PHA suspensions respectively having various amounts of organic nitrogen were prepared.

### (Example 4)

The aqueous PHA suspension prepared according to the method described above was heated while adjusting the pH of 10 with stirring. The volume mean particle diameter of PHA particles in each aqueous PHA suspension before heating was 1 µm. With respect to the aqueous PHA suspension having the amount of organic nitrogen in the aqueous PHA suspension of not less than 3,119 ppm, the heating was carried out to 80°C, but any aggregation was not observed. On the other hand, with respect to the aqueous PHA suspension having the amount of organic nitrogen lower than 3, 119 ppm, the PHA particles aggregated as the volume mean particle diameter became not less than 10 µm by heating to 80°C. The heating time period was 60 min. Thus, it was proven that as the amount of organic nitrogen in the aqueous PHA suspension is decreased, aggregation easily occurs at a temperature lower than the melting point without heating to around the melting point of PHA (about 140°C). The results of the series of studies are shown in Table 1.

Table 1: Amount of Organic Nitrogen in Aqueous PHA Suspension and Aggregability

**Table 1**

| | Amount of organic nitrogen in aqueous PHA suspension before aggregation operation, per weight of PHA (ppm) | Volume mean particle diameter | Results of aggregation |
|---|---|---|---|
| (1) | 19210 | 1 to 2 | C |
| (2) | 11018 | 1 to 2 | C |
| (3) | 6226 | 1 to 2 | C |
| (4) | 3119 | 1 to 2 | C |
| (5) | 1518 | 5 to 10 | B |
| (6) | 983 | 10 to 30 | A |
| (7) | 594 | 80 to 100 | A |
| (8) | 371 | 100 to 200 | A |

| | | | |
|---|---|---|---|
| Note the evaluations presented in the Table according to the following criteria. A: aggregation extremely proceeded enough until the volume mean particle diameter exceeded 10 µm; B: aggregation proceeded until the volume mean particle diameter fell within the range of 5 to 10 µm; C: aggregation not proceeded sufficiently, with the volume mean particle diameter being less than 5 µm. | | | |

## Claims

1. A method for producing agglomerates of poly-3-hydroxyalkanoic acid comprising:
culturing a microorganism having the ability to intracellularly produce poly-3-hydroxyalkanoic acid;
disrupting the microorganism containing the poly-3-hydroxyalkanoic acid;
purifying poly-3-hydroxyalkanoic acid by degradation and/or removal of impurities to obtain an aqueous suspension containing poly-3-hydroxyalkanoic acid having an amount of organic nitrogen in the aqueous suspension containing poly-3-hydroxyalkanoic acid adjusted to not greater than 1,500 ppm per weight of poly-3-hydroxyalkanoic acid as determined by ninhydrin reaction; and
thereafter allowing poly-3-hydroxyalkanoic acid to be aggregated in the aqueous suspension, thereby obtaining agglomerates of poly-3-hydroxyalkanoic acid.

2. The method for producing agglomerates of poly-3-hydroxyalkanoic acid according to claim 1, wherein a solvent included in the aqueous suspension containing poly-3-hydroxyalkanoic acid comprises water, an organic solvent that is miscible with water, or a mixed solvent of water and the organic solvent.

3. The method for producing agglomerates of poly-3-hydroxyalkanoic acid according to claim 1 or 2, wherein the poly-3-hydroxyalkanoic acid is a copolymer constituted with two or more types of 3-hydroxyalkanoic acid selected from the group consisting of 3-hydroxypropionate, 3-hydroxybutyrate, 3-hydroxyvalerate, 3-hydroxyhexanoate, 3-hydroxyheptanoate, and 3-hydroxyoctanoate.

4. The method for producing agglomerates of poly-3-hydroxyalkanoic acid according to claim 3, wherein the poly-3-hydroxyalkanoic acid is a binary copolymer of 3-hydroxyhexanoate and 3-hydroxybutyrate, or a ternary copolymer of 3-hydroxyhexanoate, 3-hydroxybutyrate and 3-hydroxyvalerate.

5. The method for producing agglomerates of poly-3-hydroxyalkanoic acid according to any one of claims 1 to 4, wherein the poly-3-hydroxyalkanoic acid is poly-3-hydroxyalkanoic acid produced by a microorganism that yields poly-3-hydroxyalkanoic acid.

6. The method for producing agglomerates of poly-3-hydroxyalkanoic acid according to claim 5, wherein the microorganism that yields poly-3-hydroxyalkanoic acid is a microorganism belonging to genus Aeromonas, genus Alcaligenes, genus Ralstonia, or genus Cupriavidus.

7. The method for producing agglomerates of poly-3-hydroxyalkanoic acid according to claim 6, wherein the microorganism that yields poly-3-hydroxyalkanoic acid is Cupriavidus necator.

8. The method for producing agglomerates of poly-3-hydroxyalkanoic acid according to claim 5, wherein the microorganism that yields poly-3-hydroxyalkanoic acid is a transformant into which at least one selected from a poly-3-hydroxyalkanoic acid synthase gene derived from Aeromonas caviae and a variant thereof was introduced.

9. The method for producing agglomerates of poly-3-hydroxyalkanoic acid according to any one of claims 1 to 8, wherein the amount of organic nitrogen in an aqueous suspension containing poly-3-hydroxyalkanoic acid is adjusted to not greater than 600 ppm per weight of poly-3-hydroxyalkanoic acid.

## Patentansprüche

1. Verfahren zur Herstellung von Agglomeraten der Poly-3-hydroxyalkansäure, umfassend:
Kultivieren eines Mikroorganismus mit der Fähigkeit, intrazellulär Poly-3-hydroxyalkansäure herzustellen;
Zerstören des Mikroorganismus, welcher die Poly-3-hydroxyalkansäure enthält;
Aufreinigen der Poly-3-hydroxyalkansäure durch Abbau und/oder Entfernung von Verunreinigungen, um eine wässrige, Poly-3-hydroxyalkansäure enthaltende Suspension zu erhalten, mit einer Menge an organischem Stickstoff in der wässrigen Suspension, die Poly-3-hydroxyalkansäure enthält, eingestellt auf eine Menge von nicht über 1.500 Gewichts-ppm Poly-3-hydroxyalkansäure, bestimmt durch eine Ninhydrinreaktion; und
anschließend Aggregierenlassen der Poly-3-hydroxyalkansäure in der wässrigen Suspension, wodurch Agglomerate der Poly-3-hydroxyalkansäure erhalten werden.

2. Verfahren zur Herstellung von Agglomeraten der Poly-3-hydroxyalkansäure nach Anspruch 1, wobei ein Lösungsmittel, das in die wässrige Suspension, die Poly-3-hydroxyalkansäure enthält, aufgenommen wurde, Wasser, ein organisches Lösungsmittel, das mit Wasser löslich ist, oder ein Mischlösungsmittel aus Wasser und dem organischen Lösungsmittel umfasst.

3. Verfahren zur Herstellung von Agglomeraten der Poly-3-hydroxyalkansäure nach Anspruch 1 oder 2, wobei die Poly-3-hydroxyalkansäure ein Copolymer ist, das aus zwei oder mehr Typen von 3-Hydroxyalkansäure gebildet wurde, auswählt aus der Gruppe, bestehend aus 3-Hydroxypropionat, 3-Hydroxybutyrat, 3-Hydroxyvalerat, 3-Hydroxyhexanoat, 3-Hydroxyheptanoat, und 3-Hydroxyoctanoat.

4. Verfahren zur Herstellung von Agglomeraten der Poly-3-hydroxyalkansäure nach Anspruch 3, wobei die Poly-3-hydroxyalkansäure ein binäres Copolymer von 3-Hydroxyhexanoat und 3-Hydroxybutyrat, oder ein ternäres Copolymer aus 3-Hydroxyhexanoat, 3-Hydroxybutyrat und 3-Hydroxyvalerat ist.

5. Verfahren zur Herstellung von Agglomeraten der Poly-3-hydroxyalkansäure nach einem der Ansprüche 1-4, wobei die Poly-3-hydroxyalkansäure Poly-3-hydroxyalkansäure ist, hergestellt durch einen Mikroorganismus, der Poly-3-hydroxyalkansäure produziert.

6. Verfahren zur Herstellung von Agglomeraten der Poly-3-hydroxyalkansäure nach Anspruch 5, wobei der Mikroorganismus, der die Polysäure produziert, zur Gattung Aeromonas, zur Gattung Alcaligenes, zur Gattung Ralstonia, oder zur Gattung Cupriavidus gehört.

7. Verfahren zur Herstellung von Agglomeraten der Poly-3-hydroxyalkansäure nach Anspruch 6, wobei der Mikroorganismus, der die Poly-3-hydroxyalkansäure produziert, Cupriavidus necator ist.

8. Verfahren zur Herstellung von Agglomeraten der Poly-3-hydroxyalkansäure nach Anspruch 5, wobei der Mikroorganismus, der die Poly-3-hydroxyalkansäure produziert, eine Transformante ist, in die zumindest ein Gen eingeführt wurde, ausgewählt aus einem Poly-3-hydroxyalkansäure-synthasegen, welches von Aeromonas caviae oder einer Variante hiervon abstammt.

9. Verfahren zur Herstellung von Agglomeraten der Poly-3-hydroxyalkansäure nach einem der Ansprüche 1-8, wobei die Menge an organischem Stickstoff in einer Poly-3-hydroxyalkansäure enthaltenden wässrigen Suspension so eingestellt wird, dass sie nicht über 60 Gewichts-ppm Poly-3-hydroxyalkansäure liegt.

## Revendications

1. Procédé de production d'agglomérats d'acide poly-3-hydroxyalcanoïque comprenant :
la culture d'un microorganisme ayant la capacité à produire de manière intracellulaire l'acide poly-3-hydroxyalcanoïque ;
la rupture du microorganisme contenant l'acide poly-3-hydroxyalcanoïque ;
la purification de l'acide poly-3-hydroxyalcanoïque par dégradation et/ou élimination d'impuretés pour obtenir une solution aqueuse contenant l'acide poly-3-hydroxyalcanoïque ayant une quantité d'azote organique dans la suspension aqueuse contenant l'acide poly-3-hydroxyalcanoïque ajustée à pas plus de 1500 ppm par poids d'acide poly-3-hydroxyalcanoïque comme déterminé par la réaction à la ninhydrine; et
ensuite laisser l'acide poly-3-hydroxyalcanoïque s'agréger dans la suspension aqueuse, obtenant ainsi des agglomérats d'acide poly-3-hydroxyalcanoïque.

2. Procédé de production d'agglomérats d'acide poly-3-hydroxyalcanoïque selon la revendication 1, dans lequel un solvant compris dans la suspension aqueuse contenant l'acide poly-3-hydroxyalcanoïque comprend de l'eau, un solvant organique qui est miscible avec l'eau ou un solvant mixte d'eau et du solvant organique.

3. Procédé de production d'agglomérats d'acide poly-3-hydroxyalcanoïque selon la revendication 1 ou 2, dans lequel l'acide poly-3-hydroxyalcanoïque est un copolymère constitué de deux types ou plus d'acide 3-hydroxyalcanoïque choisis dans le groupe constitué par le 3-hydroxypropionate, le 3-hydroxybutyrate, le 3-hydroxyvalérate, le 3-hydroxyhexanoate, le 3-hydroxyheptanoate et le 3-hydroxyoctanoate.

4. Procédé de production d'agglomérats d'acide poly-3-hydroxyalcanoïque selon la revendication 3, dans lequel l'acide poly-3-hydroxyalcanoïque est un copolymère binaire de 3-hydroxyhexanoate et de 3-hydroxybutyrate ou un copolymère ternaire de 3-hydroxyhexanoate, de 3-hydroxybutyrate et de 3-hydroxyvalérate.

5. Procédé de production d'agglomérats d'acide poly-3-hydroxyalcanoate selon l'une quelconque des revendications 1 à 4, dans lequel l'acide poly-3-hydroxyalcanoïque est l'acide poly-3-hydroxyalcanoïque produit par un microorganisme qui fournit l'acide poly-3-hydroxyalcanoïque.

6. Procédé de production d'agglomérats d'acide poly-3-hydroxyalcanoïque selon la revendication 5, dans lequel le microorganisme qui fournit l'acide poly-3-hydroxyalcanoïque est un microorganisme appartenant au genre Aeromonas, au genre *Alcaligenes* ou au genre *Cupriavidus.*

7. Procédé de production d'agglomérats d'acide poly-3-hydroxyalcanoïque selon la revendication 5, dans lequel le microorganisme qui fournit l'acide poly-3-hydroxyalcanoïque est *Cupriavidus necator.*

8. Procédé de production d'agglomérats d'acide poly-3-hydroxyalcanoïque selon la revendication 5, dans lequel le microorganisme qui fournit l'acide poly-3-hydroxyalcanoïque est un transformant dans lequel au moins un choisi parmi un gène d'acide poly-3-hydroxyalcanoïque synthase dérivé d'Aeromonas caviae et un de ses variants a été introduit.

9. Procédé de production d'agglomérats d'acide poly-3-hydroxyalcanoïque selon l'une quelconque des revendications 1 à 8, dans lequel la quantité d'azote organique dans une suspension aqueuse contenant l'acide poly-3-hydroxyalcanoïque est ajustée à pas plus de 600 ppm par poids d'acide poly-3-hydroxyalcanoïque.
